# EUROPÄISCHE PATENTSCHRIFT

(11) **EP 0 297 457 B1**
(45) Veröffentlichungstag und Bekanntmachung des Hinweises auf die Patenterteilung: **08.09.1993**
(21) Anmeldenummer: 88110118.2
(22) Anmeldetag: 24.06.1988
(51) Int. Cl.: A61K 7/48

(54) **Kosmetisches Mittel**
Cosmetic product
Agent cosmétique

(30) Priorität: 26.06.1987 DE 3721190
(43) Veröffentlichungstag der Anmeldung: 04.01.1989
(73) Patentinhaber: Heyl Chemisch-pharmazeutische Fabrik GmbH & Co. KG, D-14167 Berlin (DE)
(72) Erfinder: Bunte, Thomas, Dr. Dipl.Bio, Berlin 62 (DE); Parr, Wolfgang, Dr. Dipl.Chem., Berlin 38 (DE); Heyl, Eduard, Dr., Berlin 37 (DE)
(74) Vertreter: Kinzebach, Werner, Dr.

(56) Entgegenhaltungen:
- EP-A- 0 076 987

## Beschreibung

Die Erfindung betrifft ein kosmetisches Mittel, das ein Translationssystem aus einer Fraktion eines mechanisch erhaltenen Lysats aus Hefekulturen der Spezies Saccharomyces cerevisiae enthält, sowie die Verwendung dieses Translationssystems zur Behandlung von Haut.

Es ist bekannt, daß die Haut bei der Verrichtung von täglicher Arbeit sowie durch natürliche Umwelteinflüsse und durch das natürlich bedingte Altern Veränderungen erfährt. Die Haut verliert dabei an Elastizität und Flexibilität und erschlafft. Biochemisch gesehen ist dies das Resultat eines Vitalitätsverlustes der Hautzellen. Diese Zellen sind nicht mehr in der Lage, die Komponenten der extrazellulären Matrix in optimaler Form zu synthetisieren. Da es sich bei den meisten Komponenten der extrazellulären Matrix um Proteine handelt, kann man daraus schließen, daß das Protein-Biosynthesesystem der Hautzellen nicht mehr optimal funktioniert. Dies hat zur Folge, daß einerseits nicht genügend neue Komponenten des Bindegewebes synthetisiert werden können und daß andererseits nicht genügend proteolytisch wirksame Enzyme hergestellt werden, die wiederum die gealterte extrazelluläre Matrix abbauen können. Somit kann die Alterung der Haut auch unter dem Aspekt der Störung des natürlichen Protein-Turn-Overs betrachtet werden. Eine Vielzahl wissenschaftlicher Publikationen bestätigt diese Betrachtungsweise, siehe beispielsweise:
KANUNGO, M.S., D. KOUL and K.R. REDDY: Concomitant Studies on RNA and Protein Syntheses in Tissues of Rats of Various Ages. Exp. Geront. 5 (1970) 261
KAO, K.Y.T. and T.H. Mc GAVACK: Connective tissue XVIII. Age differences in protocollagen hydroxylase of porcine uterine homogenate. Proc. Soc. exper. Biol. (N.Y.) 130 (1969) 491
KAO, K. T., D. M. HILKER, T.H. Mc GAVACK: Connective tissue. III. Collagen and hexosamine content of tissues of rats at different ages. Proc. Soc. exp. Biol. 104 (1960) 359
KAO, K.T., D.M. HILKER and T.H. Mc GAVACK: Connective tissue, IV. Synthesis and turnover of proteins in tissues of rats. Proc. Soc. exp. Biol. (N.Y.) 106 (1961) 121
KAO, K.Y.T., D.M. HILKER and T.H. Mc GAVACK: Connective tissue V. Comparison of synthesis und turnover of collagen and elastin in tissues of rat of several ages. Proc. Soc. exper. Biol. (N.Y.) 106 (1961)
Aus der EP-A- 0 076 987 ist ein kosmetisches Mittel bekannt, welches neben üblichen kosmetischen Grundstoffen einen Gehalt an nativem Hefezellinhalt aufweist. Dessen hautpflegende Wirkung wird im wesentlichen dem im Mittel enthaltenen Repair-Komplex zugeschrieben. Dieser ist Bestandteil des Hefezellkerns. Soll das bekannte Mittel eine besonders hohe hautregenerierende Eigenschaft besitzen, so ist darauf zu achten, daß die verwendete Zellinhaltsfraktion den Hefezellkerninhalt möglichst quantitativ enthält.

Der vorliegenden Erfindung liegt nun die Aufgabe zugrunde, kosmetische Mittel zur Verfügung zu stellen, die die Haut revitalisieren und dem Alterungsprozeß der Haut entgegenwirken.

Diese Aufgabe wird gelöst durch ein kosmetisches Mittel, das eine Fraktion eines mechanisch erhaltenen Lysats aus Hefekulturen der Spezies Saccharomyces cerevisiae enthält, und dadurch gekennzeichnet ist, daß die Fraktion das Ausschlußvolumen ist, das man bei der Gelfiltration des Lysats und nach dem Entfernen von Zellkernen und Debris erhält.

Überraschenderweise wurde nun gefunden, daß ein zum Aufbau körpereigener Proteine befähigtes Translationssystem in einer Fraktion eines mechanisch erhaltenen Lysats von bestimmten Hefekulturen gewonnen werden kann, das die Proteinbiosynthese der Hautzellen fördert. Dabei wird der extrazelluläre Matrix-Metabolismus, der wie erwähnt, durch tägliche Arbeiten, Umwelteinflüsse und den natürlichen Alterungsprozeß gestört ist, wieder auf das physiologisch korrekte Maß gebracht, so daß die Haut revitalisiert wird.

Um das in den erfindungsgemäßen Mitteln enthaltene Translationssystem zu erhalten, werden zunächst Kulturen von Saccharomyces cerevisiae lysiert. Zu diesem Zweck zentrifugiert man Kulturen, geeigneterweise Schüttelkulturen, von Saccharomyces cerevisiae, spült sie mit physiologischer Kochsalzlösung und zentrifugiert sie erneut. Das erhaltene Sediment suspendiert man in einem HEPES*/KOH-Puffer mit neutralem pH, der außerdem Magnesiumacetat, Kaliumacetat und Sucrose enthält. Die Suspension wird anschließend bei 0 °C durch Schütteln mit Glasperlen mit einem Durchmesser von 0,05 mm lysiert. Zur völligen Lyse von 100 ml Hefesuspension ist eine Schüttelzeit von etwa 10 Minuten notwendig. Zur Lyse der Hefezellen lassen sich auch andere literaturbekannte Methoden heranziehen, wie beispielsweise die von T.G. Cooper, Biochemische Arbeitsmethoden, Walter de Gruyter, Berlin-New York 1981, Seiten 338 bis 342.
*4-(2-Hydroxyethyl)-1-piperazinethansulfonsäure.

Zweckmäßigerweise entfernt man anschließend Zellkerne und Debris durch Zentrifugation des Lysats.

Vorzugsweise handelt es sich bei der in den erfindungsgemäßen Mitteln zur Anwendung kommenden Fraktion um das Ausschlußvolumen, das man bei einer Chromatographie (Gelfiltration) des Lysats an einem Molekularsieb erhält. Für die Gelfiltration besonders geeignet ist ein quervernetztes Dextran, wie z.B. Sephadex® G-75.

Besonders bevorzugt zur Anwendung in den erfindungsgemäßen Mitteln ist eine Fraktion, die man durch weitere Auftrennung des Gelfiltrations-Ausschlußvolumens erhält. Diese Auftrennung erfolgt an einem diskontinuierlichen Gradienten (Stufengradient), vorzugsweise an einem Sucrose-Gradienten. Vorteilhafterweise beteht dieser Gradient aus folgenden Stufen: 1,8 M; 1M; 0,5 M und 0,1 M Sucrose in 30 mM Tris HCl pH 7,5; 200 mM KCl; 5 mM MgCl₂: 6 mM 2-Mercaptoethanol und 0,25 mM EDTA.

Dabei überschichtet man den diskontinuierlichen Gradienten mit dem Ausschlußvolumen der Gelfiltration und führt bei 100 000 bis 150 000 xg, vorzugsweise 130 000 xg, die Auftrennung durch. Zweckmäßigerweise arbeitet man bei Kühlraumtemperatur (0 bis 10 °C; insbesondere 4 °C). Die Zeitdauer der Auftrennung hängt ab von der Abstufung des Gradienten und der Größe der Zentrifugalkraft; sie beträgt im allgemeinen 10 bis 30 Stunden, zweckmäßigerweise 15 bis 20 Stunden. In den einzelnen Stufen des Gradienten befinden sich dann die in der Dichte entsprechenden Fraktionen des Ausschlußvolumens. Unterfraktionen können dann noch durch Auftrennung der einzelnen Stufen des Gradienten in mehrere Schichten gewonnen werden.

Die Fraktionen kann man als solche, das heißt als Lösung in standardisierter Form oder vorzugsweise als Lyophilisat anwenden. Die Lyophilisierung wird nach üblichen, dem Fachmann bekannten Verfahren durchgeführt.

Zur Anwendung in den erfindungsgemäßen Mitteln kann man eine oder mehrere der Fraktionen verwenden, die sich in den einzelnen Stufen des Gradienten angereichert haben. Ebenfalls geeignet sind die schwersten Polyribrosomen, die sich nicht in einer der Stufen des Gradienten, sondern am Boden des Zentrifugenglases sammeln. Besonders bevorzugt ist diejenige Fraktion, die sich in der 1,8 M-Stufe des oben erwähnten Sucrosegradienten befindet.

Vorzugsweise enthalten die erfindungsgemäß zur Anwendung kommenden Fraktionen keine endogene m-RNA. Soweit vorhanden, kann diese durch Inkubieren der Fraktion mit Mikrococcusnuclease beseitigt werden.

Die in den erfindungsgemäßen Mitteln zur Anwendung kommenden Fraktionen enthalten das gesamte ribosomale Proteinbiosynthesesystem sowie gegebenenfalls t-RNA, ATP, GTP und für die Proteinbiosynthese übliche Aminosäuren. Die letzteren Bestandteile können gewünschtenfalls auch zugesetzt werden. Außerdem kann man weitere Bestandteile, wie Mineralstoffe, Spurenelemente, Vitamine und andere Wirkstoffe zugeben. Die erfindungsgemäßen Fraktionen enthalten, wie erwähnt, ribosomale Aktivität und sind in der Lage, exogene m-RNA zu lesen und zu translatieren. Man nimmt an, daß ihre Wirkung darin besteht, daß sie funktionsfähige ribosomale Aktivität bereitstellen, die geschädigte Ribosomen ersetzt. Damit stehen wieder ausreichend Ribosomen für die Translation von Aminosäuren in Proteine zur Verfügung, so daß die Proteinbiosynthese geschädigter bzw. gealteter Haut gefördert wird.

Erfindungsgemäß geeignet sind Fraktionen, die in der Lage sind, exogene m-RNA zu lesen und in Protein zu translatieren. Sie sind außerdem definiert durch ihr Sedimentationsverhalten im Dichtegradienten und durch die Zusammenarbeit der Proteine in der Fraktion. Diese Eigenschaften werden bestimmt wie von B. Schulz-Harder und E.R. Lochmann in Zeitschrift für Naturforschung 31c, 169 - 173 (1976) beschrieben.

Die erfindungsgemäß zur Anwendung kommenden Fraktionen werden anhand ihrer Fähigkeit, ³⁵S- Methionin in Protein zu inkorporieren, standardisiert. Dies geschieht folgendermaßen:
Zu 0,5 ml der Fraktion gibt man 1µg exogene mRNA, 2 mC ³⁵S-Methionin und die übrigen nicht-radioaktiven Aminosäuren. Dieses System wird anschließend 30 Minuten bei 37 °C inkubiert. Man gibt dann das dreifache Volumen (1,5 ml) 10%iger Trichloressigsäure zur Fällung des Proteins zu und inkubiert 10 Minuten bei 4 °C. Das Protein wird abfiltriert und getrocknet. Anschließend bestimmt man die in dem gefällten Protein vorhandene Radioaktivität im Szintillationszähler (siehe B. Schultz-Harder und E.R. Lochmann, ibid).

Man stellt die Fraktionen dann so ein, daß sie gemäß der obigen Standardisierungsmethode im Szintillationszähler 3000 - 5000 counts/Min. liefern.

Die auf diese Weise eingestellten Fraktionen inkorporiert man in die kosmetischen Mittel als Lösung in einer Menge von 3 bis 40 Vol-%, vorzugsweise 5 bis 20 Vol-%, oder als Lyophilisat in der entsprechenden Menge.

Anhand von in vitro und in vivo-Versuchen konnte gezeigt werden, daß die natürliche Proteinbiosyntheserate bei Applikation der erfindungsgemäßen Mittel erheblich gesteigert werden kann. Diese stimulierende Wirkung der erfindungsgemäßen Mittel wurde mit den folgenden Versuchen nachgewiesen:
1. In vitro-Tests an:
   a) einer Ovarialzellinie des chinesischen Hamsters;
   b) menschlichen Fibroblasten.
   Die Biosynthesekapazität der erwähnten Zellen wurde durch Zugabe von Cycloheximid vollständig blockiert. Die Zellen wurden dann in zwei Teile geteilt. Der eine Teil wurde zusammen mit der erfindungsgemäß zur Anwendung kommenden Fraktion und mit der radioaktiven Aminosäure ³⁵S - Methionin inkubiert. Der andere Teil wurde ebenfalls zusammen mit ³⁵S - Methionin,aber ohne die erwähnte Fraktion inkubiert.
   Während der Inkubation erfolgte eine Proteinbiosynthese, bei der ³⁵S - Methionin in die Proteine eingebaut wurde. Nach dem Abschluß der Inkubation wurden die Proteine mit Säure gefällt und das gefällte Protein auf das Vorhandensein von Radioaktivität getestet.
   Es wurde gefunden, daß der mit der erfindungsgemäß zur Anwendung kommenden Fraktion behandelte Ansatz gegenüber dem Kontrollansatz eine hochsignifikante Proteinbiosyntheserate zeigte.
2. In vivo-Tests an Mäusen:
   Den Tieren wurde auf rasierten Arealen des Rückens zunächst Cycloheximid intrakutan gespritzt, um die Proteinbiosynthese der Haut zu inhibieren. Anschließend wurde einer Gruppe der Mäuse eine kosmetische Zusammensetzung, die nur aus dem Träger bestand, zusammen mit ³⁵S - Methionin und einer anderen Gruppe der Mäuse die gleiche kosmetische Zusammensetzung, die jedoch 15 % der erfindungsgemäßen Fraktion enthielt, zusammen mit ³⁵S - Methionin in jeweils einer Menge von 0,1 g aufgetragen.
   Nach einer Inkubationszeit von 12 Stunden tötete man die Mäuse mit Äther und entfernte die Hautareale. Man extrahierte die Hautareale und bestimmte anschließend die Radioaktivität in den Proteinen, die mit Säure aus dem Extrakt gefällt werden konnten.
   Es hat sich gezeigt, daß die Zusammensetzung mit der erfindungsgemäßen Fraktion eine Proteinbiosynthese der Haut trotz Inaktivierung der Haut-Proteinbiosynthese durch Cycloheximid ermöglicht. Das bedeutet, daß die erfindungsgemäßen Mittel in der Lage sind, die Proteinbiosynthese der Hautzellen zu steigern.

Die erfindungsgemäßen Mittel werden lokal (topisch) verabreicht. Sie kommen hierfür in üblichen Formulierungen, wie Creme, Salbe, Gel, Lotion, Lösung oder Gesichtsmaske zur Anwendung, in die sie als Lösung oder auch als Lyophilisat in den oben angegebenen Mengen eingearbeitet werden können. Diese Formulierungen basieren auf üblichen Trägersystemen, wie Polyäthylenglykole , Carboxymethylcellulose, Carboxyvinylpolymerisate, Paraffinöle, Cetylstearylalkohol, Ölsäureester, Fettsäuretriglyceride, Polyacrylate, Glycerin, Alkohole und dergleichen. Sie können auch üblicherweise verwendete Hilfsmittel enthalten, wie Konservierungsmittel, Parfümöle, Puffer, Netzmittel und dergleichen.

Die nachfolgenden Beispiele erläutern die Erfindung.

Die Prozentangaben bedeuten Volumenprozent.

### Beispiel 1

### Herstellung einer W/O-Creme mit folgenden Bestandteilen:

a)

| | |
|---|---|
| Cetylstearylalkohol | 9,0 % |
| Natriumcetylstearylsulfat | 1,0 % |
| Ölsäuredecylester | 10,0 % |
| Wollfett | 2,0 % |
| Triglycerid von fraktionierten Kokosölfettsäuren | 5,0 % |
| Konservierungsmittel (Phenonip ¹⁾) | 0,3 % |

| | |
|---|---|
| ¹⁾flüssiges Konservierungsmittel auf Basis eines Gemisches von p-Hydroxybenzoesäureestern und Phenoxyethanol | |

b)

| | |
|---|---|
| destilliertes Wasser | 57,0 % |
| 70%ige Lösung von Sorbit | 5,0 % |

c)

| | |
|---|---|
| Proteinbiosynthese Wirkstoffsystem: Ausschlußvolumen der Gelfiltration (oder das entsprechende Lyophilisat) | 10,0 % |

Die Zubereitung des Mittels erfolgt in der Weise, daß zunächst die Ingredienzienkombination a) geschmolzen und auf ca. 70 °C erwärmt und die erhaltene Schmelze die unter b) angeführte, auf 70 °C erwärmte Lösung eingerührt wird. Das Ganze wird weitergerührt, bis eine Abkühlung der Creme auf ca. 35 °C eingetreten ist. Danach wird die Komponente c) eingerührt, bis sie gleichmäßig verteilt ist.

Anstelle des bei der Gelfiltration erhaltenen Ausschlußvolumens kann man auch eine der Fraktionen verwenden, die man bei der Auftrennung des Ausschlußvolumens an einem diskontinuierlichen Sucrose-Gradienten erhält.

### Beispiel 2

### Herstellung einer W/O-Creme mit folgenden Bestandteilen:

a) Gemisch aus hochmolekularen Estern, vorwiegend Mischester aus Pentaerythrit-Fettsäureester und Citronensäurealkoholester und mineralischen Fetten

| | |
|---|---|
| Ölsäuredecylester | 20,0 % |
| Pflanzenöl | 5,0 % |
| weiße Vaseline | 5,0 % |
| Konservierungsmittel | q.s. |

b)

| | |
|---|---|
| destilliertes Wasser | 55,0 % |

c)

| | |
|---|---|
| Proteinbiosynthese | 15 % |

Wirkstoffsystem: In der 1,8 M Stufe des diskontinuierlichen Sucrosegradienten enthaltene Fraktion (oder das entsprechende Lyophilisat)
Die Zubereitung des Mittels erfolgt nach der in Beispiel 1 angegebenen Methode.

### Beispiel 3

### Herstellung eines Gels mit folgenden Bestandteilen:

a)

| | |
|---|---|
| Wasser, destilliert, konserviert | 50,0 % |
| Carbopol 40 (Polyacrylsäure) | 0,5 % |
| Phenonip (Konservierungsmittel) | 0,3 % |

b)

| | |
|---|---|
| Wasser, destilliert, konserviert | 28,2 % |
| Triaethanolamin | 1,0 % |

c)

| | |
|---|---|
| Proteinbiosynthese Wirkstoffsystem: | 20,0 % |

In der 1,8 M Stufe des diskontinuierlichen Succrosegradienten enthaltene Fraktion (oder das entsprechende Lyophilisat)
Die Zubereitung des Mittels erfolgt in der Weise, daß die Ingredenzien-Kombination a) durch schnelles Rühren dispergiert wird. Danach wird die unter b) angeführte Lösung und anschließend die Komponente c) eingerührt.

### Beispiel 4

### Herstellung einer Lotion mit folgenden Bestandteilen:

a)

| | |
|---|---|
| Äthanol 96 vol.-% | 15,0 % |

b)

| | |
|---|---|
| Wasser, destilliert, konserviert | 76,4 % |
| Zitronensäure | 0,3 % |
| Phenonip | 0,3 % |
| 1,2 Propylenglykol | 3,0 % |

c)

| | |
|---|---|
| Proteinbiosynthese Wirkstoffsystem: | 5,0 % |

In den 1M und 0,5 MStufen des diskontinuierlichen Succrosegradienten enthaltene Fraktion(oder das entsprechende Lyophilisat).

Die Zubereitung des Mittels erfolgt in der Weise, daß aus den Ingredienzien unter b) eine Lösung hergestellt wird. Danach wird zuerst die Komponente a) und anschließend die Komponente c) eingerührt.

### Beispiel 5

### Herstellung einer Gesichtsmaske mit folgenden Bestandteilen:

| | |
|---|---|
| autoemulgierendes Glycerinmonostearat | 26 g |
| Reisstärke | 18 g |
| Kaolin | 35 g |
| Mandelkleie | 10 g |

### Proteinbiosynthese Wirkstoffsystem:

Lyophilisat von 30 ml des Ausschlußvolumens der Gelfiltration oder der in der 0,5; 1 oder 1,8 M Stufe des diskontinuierlichen Sucrosegradienten enthaltenen Fraktion.

Das Glycerinmonostearat wird pulverisiert und mit den anderen Bestandteilen vermischt. Bei der Anwendung wird die Mischung mit etwa 400 ml heißem Wasser zu einem cremigen Teig angerührt und bei erträglichen Temperaturen auf die Haut aufgetragen.

Die in den Beispielen 1 bis 5 verwendeten Fraktionen waren auf eine Proteinmenge eingestellt, die gemäß obigen Standardisierungsverfahren 4000 counts/min entspricht.

### Beispiel 6

### Herstellung der erfindungsgemäßen Fraktionen:

Schüttelkulturen von Saccharomyces cerevisiae werden zentrifugiert, mit physiologischer Kochsalzlösung abgespült, erneut zentrifugiert und das Sediment in einem Puffer, bestehend aus 30 mM Tris HCl pH 7,5; 200 mM KCl; 5 mM MgCl₂; 500 mM Saccharose; 6 mM 2 Mercaptoethanol; 0,25 mM EDTA; 20 % V:V resuspendiert wird. Die resuspendierte Lösung wird anschließend bei 0 °C durch Schütteln mit Glasperlen eines Durchmessers von 0,05 mm lysiert. Um 100 ml der Hefesuspension zu lysieren, sind 10 Min. Schütteln notwendig. Das so gewonnene Homogenat wird 10 Min. lang bei 18.000 g zentrifugiert, um Zellkerne und zelluläres Debris zu entfernen. Der gereinigte Überstand wird über eine Sephadex® G 75 Molekularsiebsäule gegeben. Das Ausschlußvolumen wird gesammelt und kann für die erfindungsgemäßen Mittel verwendet werden.

Das gesamte Ausschlußvolumen wird anschließend auf 1 % nichtionisches Detergens wie z.B., Brij® 58 eingestellt und anschließend einem diskontinuierlichen Sucrosegradienten überschichtet. Dieser Gradient besteht aus folgenden Stufen: 1,8 M: 1M; 0,5 M und 0,1 M Sucrose in 30 mM Tris HCl pH 7,5; 200 mM KCl; 5 mM MgCl₂; 6 mM 2-Mercaptoethanol und 0,25 mM EDTA. Der Ansatz wird in einem Schwingbecherrotor mit 130.000 xg für 18 Std. bei 4 °C zentrifugiert. Der Gradient wird anschließend fraktioniert. Die einzelnen Fraktionen können für die erfindungsgemäßen Mittel verwendet werden.

Falls gewünscht, können die Fraktion nach üblichen Verfahren lyophilisiert werden.

## Patentansprüche

1. Kosmetisches Mittel, enthaltend ein Translationssystem, bestehend aus einer Fraktion eines mechanisch gewonnenen Lysats aus Hefekulturen der Spezies Saccharomyces cerevisiae,
**dadurch gekennzeichnet**, daß
die Fraktion das Ausschlußvolumen ist, das man bei der Gelfiltration des Lysats nach Entfernen von Zellkernen und Debris erhält.

2. Mittel nach Anspruch 1, wobei die Fraktion des Lysates aus Hefekulturen der Spezies Saccharomyces cerevisiae dadurch erhältlich ist, daß man
a) Saccharomyces cerevisiae züchtet, die gewachsene Kultur abzentrifugiert, spült und erneut zentrifugiert;
b) das Sediment aus Schritt a) in einem Puffer mit neutralem pH suspendiert und die suspendierten Zellen mechanisch lysiert;
c) aus dem Lysat Zellkerne und Debris entfernt;
d) das Lysat aus Schritt c) gelfiltriert; und
e) das Ausschlußvolumen der Gelfiltration isoliert.

3. Mittel nach einem der Ansprüche 1 oder 2,
dadurch gekennzeichnet, daß die Gelfiltration an quervernetztem Dextran (Sephadex® G-75) erfolgt.

4. Mittel nach einem der Ansprüche 1 bis 3,
dadurch gekennzeichnet, daß die Fraktion eine Fraktion ist, die erhältlich ist durch Fraktionierung des Ausschlußvolumens der Gelfiltration an einem diskontinuierlichen Sucrosegradienten aus 1,8 M; 1M; 0.5 M und 0,1 M Sucrose in 30 mM Tris HCl pH 7,5; 200 mM KCl; 5 mM MgCl₂; 6 mM 2-Mercaptoethanol und 0,25 mM EDTA.

5. Mittel nach Anspruch 4,
dadurch gekennzeichnet, daß die Fraktion aus der 1,8 M-Stufe des Gradienten stammt.

6. Mittel nach einem der vorhergehenden Ansprüche,
dadurch gekennzeichnet, daß die Fraktion in Form eines Lyophilisates vorliegt.

7. Verwendung des Fraktionssystems gemäß einem der Ansprüche 1 bis 6 zur Herstellung eines kosmetischen Mittels zur Behandlung von Haut.

## Claims

1. Cosmetic agent containing a translation system consisting of a fraction of a mechanically obtained lysate from yeast cultures of the species Saccharomyces Cerevisiae, characterized in that the fraction is the exclusion volume which is obtained during gel filtration of the lysate after removal of cell nuclei and debris.

2. Agent according to claim 1, in which the fraction of the lysate from yeast cultures of the species Saccharomyces Cerevisiae can be obtained by
a) culturing Saccharomyces Cerevisiae, centrifuging the culture after growth, rinsing it and centrifuging it again;
b) suspending the sediment from step a) in a buffer having a neutral pH and mechanically lysing the suspended cells;
c) removing cell nuclei and debris from the lysate;
d) gel-filtering the lysate from step c); and
e) isolating the exclusion volume obtained in the gel filtration.

3. Agent according to claim 1 or 2, characterised in that the gel filtration is carried out on crosslinked dextran (Sephadex ® G-75).

4. Agent according to one of claims 1 to 3, characterised in that the fraction is a fraction which can be obtained by fractionating the exclusion volume from the gel filtration on a discontinuous sucrose gradient consisting of 1.8M; 1M; 0.5M and 0.1M sucrose in 30mM Tris HCl pH 7.5; 200 mM KCl; 5mM MgCl₂; 6mM 2-mercaptoethanol and 0.25mM EDTA.

5. Agent according to claim 4, characterized in that the fraction comes from the 1.8M stage of the gradient.

6. Agent according to one of the preceding claims, characterized in that the fraction takes the form of a lyophilisate.

7. Use of the fraction system according to one of claims 1 to 6 for the preparation of a cosmetic agent for treating the skin.

## Revendications

1. Agent cosmétique contenant un système de translation consistant en une fraction d'un lysat obtenu mécaniquement à partir de cultures de levure de l'espèce Saccharomyces cerevisiae, caractérisé en ce que la fraction est le volume d'exclusion que l'on obtient lors de la filtration sur un gel du lysat, après élimination des noyaux cellulaires et débris.

2. Agent suivant la revendication 1, dans lequel la fraction du lysat de cultures de levure de l'espèce Saccharomyces cerevisiae peut être obtenue de la façon suivante :
a) on cultive des Saccharomyces cerevisiae, on centrifuge la culture cultivée, on la rince et la centrifuge à nouveau,
b) on met en suspension le sédiment de l'étape a) dans un tampon à pH neutre et on lyse mécaniquement les cellules mises en suspension.
c) on élimine du lysat les noyaux cellulaires et débris,
d) on filtre sur un gel le lysat de l'étape c), et
e) on isole le volume d'exclusion de la filtration sur un gel.

3. Agent suivant l'une des revendications 1 ou 2, caractérisé en ce que la filtration sur un gel se fait sur du dextran réticulé (Sephadex® G-75).

4. Agent suivant l'une des revendications 1 à 3, caractérisé en ce que la fraction est une fraction qui peut être obtenue par fractionnement du volume d'exclusion de la filtration sur un gel avec un gradient de saccharose discontinu de 1,8 M, 1 M, 0,5 M et 0,1 M de saccharose dans 30 mM de Tris-HCL, pH 7,5, 200 mM de KCl, 5 mM de MgCl₂, 6 mM de 2-mercaptoéthanol et 0,25 mM d'EDTA.

5. Agent suivant la revendication 4, caractérisé en ce que la fraction provient de l'étage 1,8 M du gradient.

6. Agent suivant l'une des revendications précédentes, caractérisé en ce que la fraction se présente sous forme d'un lyophilisat.

7. Utilisation du système de fraction suivant l'une des revendications 1 à 6 pour la préparation d'un agent cosmétique pour le traitement de la peau.
